(19) Europäisches Patentamt

European Patent Office

Office européen des brevets

(11) **EP 1 533 384 A1**

(12) **EUROPEAN PATENT APPLICATION**
published in accordance with Art. 158(3) EPC

(43) Date of publication:
**25.05.2005 Bulletin 2005/21**

(51) Int Cl.⁷: **C12Q 1/527**, G01N 33/50, G01N 33/15, C12C 1/16

(21) Application number: **03760929.4**

(22) Date of filing: **20.06.2003**

(86) International application number:
**PCT/JP2003/007887**

(87) International publication number:
**WO 2004/001066 (31.12.2003 Gazette 2004/01)**

(84) Designated Contracting States:
**AT BE BG CH CY CZ DE DK EE ES FI FR GB GR HU IE IT LI LU MC NL PT RO SE SI SK TR**

(30) Priority: **20.06.2002 JP 2002180315**

(71) Applicant: **SAPPORO BREWERIES LTD.**
**Tokyo 150-8686 (JP)**

(72) Inventors:
• **KURODA, Hisao, c/o Sapporo Breweries Limited**
**Yaizu-shi, Shizuoka 425-0013 (JP)**

• **FURUSHO, Shigeki,**
**c/o Sapporo Breweries Limited**
**Yaizu-shi, Shizuoka 425-0013 (JP)**
• **KOJIMA, Hidetoshi,**
**c/o Sapporo Breweries Limited**
**Yaizu-shi, Shizuoka 425-0013 (JP)**

(74) Representative: **VOSSIUS & PARTNER**
**Siebertstrasse 4**
**81675 München (DE)**

(54) **METHOD OF SCREENING MALT AND PROCESS FOR PRODUCING FOAMING MALT BEVERAGE**

(57)     A method for screening malt **characterized by** evaluating fatty acid hydroperoxide lyase activity in malt, and a method for producing a malt-based sparkling beverage **characterized by** using malt having low fatty acid hydroperoxide lyase activity screened by the method for screening.

**EP 1 533 384 A1**

## Description

## Technical Field

The present invention relates to a method for screening malt and a method for producing a malt-based sparkling beverage using malt screened by the same.

## Background Art

Malt, the raw materials for malt-based sparkling beverages such as beer and low malt beer, contains large amounts of lipids and fatty acids. These lipids and fatty acids are auto-oxidized, or are oxidized by lipoxygenases, during the brewing stage of the production of malt-based sparkling beverages, to yield lipid hydroperoxides and fatty acid hydroperoxides (Kobayashi, N., Kaneda, H., Kano, Y., and Koshino, S., J. Ferment. Bioeng., 76, 371-375, 1993). Lipases contained in the malt hydrolyze these lipid hydroperoxides into fatty acid hydroperoxides. Through thermal or chemical degradation, these fatty acid hydroperoxides generate degradation products such as aldehyde having a stale, grassy, fatty acid taste, etc., which greatly impairs the flavor of the product (malt-based sparkling beverage) (Drost, B. W., van Eerde, P., Hoekstra, S. F., and Strating, J., Proc. of the 13th Congress, Estoril, Portugal, 1971).

Conventional technologies proposed for curbing the generation of these degradation products include methods for inhibiting lipoxygenase activity by increasing the temperature in the brewing stage of the manufacturing process of the malt-based sparkling beverage, or methods where the malt used has a low lipoxygenase activity (Drost, B. W., van den Berg, R., Freijee, F. J.M., van der Velde, E. G., and Hollemans, M., J. Am. Soc. Brew. Chem., 48, 124-131, 1990).

However, such methods based on increasing the brewing temperature are problematic in that they inhibit as well the action of proteases and diastatic enzymes, resulting in an insufficient nutrient supply for fermentation that detracts from the quality of flavor. These methods have therefore limitations as regards inhibition of stale flavor. Similarly, methods using malt having a low lipoxygenase activity are also imperfect, since here malt is treated at high temperatures in order to inactivate lipoxygenase, which affects negatively the quality of flavor disrupting among others the flavor balance. Furthermore, lipid hydroperoxides are also known to be already present in malt. These lipid hydroperoxides are hydrolyzed into fatty acid hydroperoxides by lipases in the fermentation mash and generate degradation products without lipoxygenase intervening in the process; thus the approach of improving flavor quality by inhibiting lipoxygenase has limitations as well (Kobayashi, N., Kaneda, H., Kano, Y., and Koshino, S., J. Am. Soc. Brew. Chem., 52(4): 141-5, 1994).

## Disclosure of the Invention

In light of the above problems of conventional technology, an object of the present invention is to provide a method for screening malt useful for manufacturing malt-based sparkling beverages having a reduced stale flavor (material selection method), and a method for manufacturing malt-based sparkling beverages using malts screened on the basis of such a malt screening method.

As a result of diligent research toward achieving the above purpose, the present inventors discovered first that enzymes were also involved in the process wherein fatty acid hydroperoxides are broken into degradation products (aldehyde, etc.). Specifically, the present inventors discovered that in the manufacturing process of malt-based sparkling beverages, fatty acid hydroperoxide lyases degrade and cleave fatty acid hydroperoxides generating degradation products. The present inventors also found out that fatty acid hydroperoxide lyases have a high thermal stability and, therefore, it is difficult to inhibit the action of these enzymes in conventional methods that rely on increasing the brewing temperature. Accordingly, the inventors perfected the present invention upon discovering that by screening malts having a low fatty acid hydroperoxide lyase activity and using them as the raw materials, the generation of degradation products (staleness substances) could be inhibited, thus making it possible to manufacture a malt-based sparkling beverage with an improved flavor quality.

Specifically, the method for screening malt according to the present invention is characterized by measuring fatty acid hydroperoxide lyase activity in malt.

In the method for screening according to the present invention, the fatty acid hydroperoxide lyase activity may be evaluated by measuring (i) the amount of degradation products generated upon degradation of fatty acid hydroperoxides by fatty acid hydroperoxide lyases, or (ii) by measuring the decreased amount of fatty acid hydroperoxides due to degradation of fatty acid hydroperoxides by fatty acid hydroperoxide lyases.

In addition, the method for producing malt-based sparkling beverages according to the present invention is characterized by using malts with low fatty acid hydroperoxide lyase activity, wherein these malts are screened by the above screening method according to the present invention.

**Brief Description of the Drawings**

Fig. 1 is a schematic flow chart illustrating the steps in experimental numbers 1 to 4 in SPME-GC-MS analysis in Verification test 1.

Fig. 2 is a graph showing the amounts of generated degradation products (trans-2-nonenal) obtained in the experimental numbers 1 to 4 in Verification test 1.

Fig. 3 is a graph showing the time course of mash temperature and fatty acid hydroperoxide lyase activity, obtained by measuring the time course of fatty acid hydroperoxide lyase activity in Verification test 2.

Fig. 4 is a graph showing the amounts of generated degradation products (trans-2-nonenal) in the experimental numbers 1 to 4, obtained in Verification test 3.

Fig. 5 is a graph showing the correlation between the nonenal potentials and the 9-linoleic acid hydroperoxide lyase activities, obtained in Example 4.

**Best Modes for Carrying Out the Invention**

Preferred embodiments of the present invention are described in detail below.

(Method for screening malt)

The method for screening malt according to the present invention is a method for evaluating fatty acid hydroperoxide lyase activity in malts. In the present invention, fatty acid hydroperoxide lyases are enzymes that degrade fatty acid hydroperoxides. Fatty acid hydroperoxides are generated from lipids and fatty acids present in malt, for instance, linoleic acid hydroperoxide (9-linoleic acid hydroperoxide (9-HPOD), 13-linoleic acid hydroperoxide (13-HPOD)), 9-linolenic acid hydroperoxide and 13-linolenic acid hydroperoxide. These fatty acid hydroperoxides is also available from Larodan Fine Chemicals, Malmo, Sweden, etc.

The present inventors discovered first that such fatty acid hydroperoxide lyases are involved in degradation of fatty acid hydroperoxides in the manufacturing process of malt-based sparkling beverages.

In the present invention, fatty acid hydroperoxide lyase activity can be suitably evaluated on the basis of the following methods (i) and (ii). (i) Setting a predetermined amount of fatty acid hydroperoxides in a malt extract (for instance, by adding fatty acid hydroperoxides to the malt extract), incubating then the extract under specific conditions (for instance, 15 minutes at 20°C), and measuring the amount of degradation products generated upon degradation of the fatty acid hydroperoxides by fatty acid hydroperoxide lyases. (ii) Setting a predetermined amount of fatty acid hydroperoxides in a malt extract (for instance, by adding fatty acid hydroperoxides to the malt extract), incubating then the extract under specific conditions, and measuring the decrease in the amount of fatty acid hydroperoxides as a result of their degradation by fatty acid hydroperoxide lyases. Such malt extracts can be obtained by adding a predetermined amount of milled malt to a predetermined amount of a buffer solution (for instance, acetic acid buffer solution) and then stirring for a predetermined time. The above degradation products are generated by the degradation of fatty acid hydroperoxides, and include for instance aldehydes, etc., specifically nonenal (trans-2-nonenal), hexanal, hexenal, nonandienal, etc.

The methods used for measuring the amount of generated degradation products are not particularly restricted, and may include for instance a method of derivatizing degradation products using derivatization reagents and assaying them by high-performance liquid chromatography (HPLC), a method of assaying the degradation products by gas chromatography. The methods used for measuring the decreased amount of fatty-acid hydroperoxides are not particularly restricted, and may include conventional methods, for instance measuring the amount of decrease of fatty acid hydroperoxides as a substrate by UV absorption.

Fatty acid hydroperoxide lyase activity is lower when the generation of degradation products is smaller or proceeds at a slower rate, or when the decrease in fatty-acid hydroperoxides is smaller or proceeds at a slower rate. Fatty acid hydroperoxide lyase activity can therefore be evaluated using the formulae below on the basis of the values measured by the above methods.

(i) In the case that the amount of generated degradation products (aldehydes, etc.) is measured, fatty acid hydroperoxide lyase activity (enzyme activity) is calculated using the formula below:

$$\text{Enzyme activity (mU/g)} = \text{amount of degradation}$$

product generated per minute (μM) x total reaction

solution (mL) ÷ enzyme solution (mL) ÷ enzyme

solution concentration (g/mL).

(ii) In the case that the decreased amount of fatty acid hydroperoxides is measured by UV absorption, the enzyme activity is given by the formula below:

Enzyme activity (nkat/g) = UV absorption decrease per

minute at 234 nm x 0.667 x total reaction solution

(mL) ÷ enzyme solution (mL) ÷ enzyme solution

concentration (g/mL).

Malts with low fatty acid hydroperoxide lyase activity can be assessed using such activity measurements; herein malts having lower fatty acid hydroperoxide lyase activity can be preferably screened, specifically, malts with an enzymatic activity of 2 mU/g or less, more preferably 0.1 mU/g or less; or 5 nkat/g or less, more preferably 1 nkat/g or less. The detection limits in the above methods are 0.1 mU/g for the measurement of the amount of degradation products (aldehydes), and 1 nkat/g for the measurement of the decrease of fatty acid hydroperoxides by UV absorption.

In the present invention, measuring fatty acid hydroperoxide lyase activity, which is correlated to wort nonenal potential, allows predicting the staleness of the final product. Product staleness refers herein to deterioration in the quality of accompanied by the storage of final products filled in containers. The wort nonenal potential is an index that allows predicting product staleness, and is measured on wort prepared by the congress method (European Brewery Convention. Analytica-EBC (5th ed.), 1998) using the method proposed by Drost et al. (Drost, B. W., van den Berg, R., Freijee, F. J.M., van der Velde, E. G., and Hollemans, M., J. Am. Soc. Brew. Chem., 48, 124-131, 1990). In order to manufacture malt-based sparkling beverages with superior staleness resistance, the raw materials malts have preferably wort nonenal potentials of 10 ppb or less, more preferably 1 ppb or less.

(Method for producing malt-based sparkling beverages)

Malts having low fatty acid hydroperoxide lyase activity as determined by the screening method of the present invention are used as raw materials in a method for producing malt-based sparkling beverages according to the present invention.

Such a producing method according to the present invention may include ordinary manufacturing processes under normal conditions, such as a mashing step, a wort boiling step, a cooling step, a fermentation step, a maturation step, etc., without restriction to any specific set of steps.

In the mashing step, the raw materials including malt are saccharified to yield a mash liquid. The malts used in the present invention are malts having low fatty acid hydroperoxide lyase activity as determined by the screening method of the present invention, specifically malts with an enzyme activity of 2 mU/g or less, more preferably 0.1 mU/g or less; or 5 nkat/g or less, more preferably 1 nkat/g or less. Preferred such malts are obtained from barley germinated under provision of sufficient moisture and air, followed by drying and germ removal. Malt is the source of enzymes necessary for wort production as well as the main starch source in the mash. In addition, malt kilning imparts to malt-based sparkling beverages their characteristic flavor and color. The target malt may be obtained for instance by steeping barley to a steeping degree of 40 to 45%, with germination for 3 to 6 days at 10 to 20°C, followed by roasting.

The methods for mashing the raw materials including malt are not particularly restricted, and may involve mixing in a brewing tank the raw materials including malt and water for brewing, then heating this mixture at a predetermined temperature (preferably 65 to 75°C) to obtain a mashing liquid, from which solids are removed by filtration as needed. The usage ratio of malt in the raw materials is selected in accordance with the intended type of malt-based sparkling beverages such as beer and low-malt beer. Herein commercially available or separately prepared malt extracts can be mixed with the brewing water, while corn starch, corn grits, rice, sugars, and etc. may be also added as adjunct materials.

In the wort boiling step, hops are added to the wort obtained by filtration of the mash liquid, and the mixture is then boiled. The malt-based sparkling beverage acquires thereby its characteristic bitter taste while at the same time the action of the malt enzymes is stopped. The amount of hops in the mash liquid ranges preferably from 0.5 to 3.0 g/ L, and the boiling time of the corresponding mixture ranges preferably from 90 to 120 minutes.

The wort after the wort boiling step (hot wort) is cooled down to a predetermined temperature, and passes then to the fermentation step described below. In this cooling step, the hot wort is ordinarily cooled to 15°C or less.

In the fermentation step, yeasts are added to the cooled wort in order to allow the wort to ferment; consequently fermentation liquid is obtained. The yeasts used in the fermentation step are not particularly restricted provided they generate alcohol and/or carbon dioxide by metabolizing sugars in the wort (alcoholic yeasts for alcoholic fermentation), and include specifically Saccharomyces cerevisiae, Saccharomyces uvarum, etc.

The fermentation conditions are not particularly restricted, but the fermentation temperature is preferably 15°C or less, and ranges yet more preferably from 8 to 10°C, while the fermentation time ranges preferably from 8 to 10 days. The fermentation liquid thus obtained is then matured, after which it is filtered to yield a malt-based sparkling beverage.

The conditions of the maturation step are not particularly limited, and may involve for instance storage in airtight storage tanks at a storage temperature of -5 to 3°C for 30 to 90 days in order to allow an adequate re-fermentation and maturation of the remaining extract.

The filtering conditions are not particularly restricted, and may include using filtering aids such as diatomaceous earth, polyvinyl polypyrrolidone (PVPP), silica gel, cellulose powder, and etc. The filtered malt-based sparkling beverage can be shipped to market in tanks, kegs, bottles, cans, and etc.

The malt-based sparkling beverages that may be produced by the method according to the present invention are, for example, beer and low-malt beer. Furthermore, the malt-based sparkling beverages that are manufactured by the method for producing according to the present invention have an excellent staleness resistance due to a reduced content in staleness substances such as aldehydes and the like caused by degradation of fatty acid hydroperoxides in the malt.

[Examples]

Examples of the present invention are described in detail below, though the invention is in no way meant to be limited to or by them. The following tests were carried out in order to verify that fatty acid hydroperoxide lyases are the enzymes that degrade fatty acid hydroperoxides into degradation products.

(Verification test 1)

Verification that fatty acid hydroperoxide lyases are active enzymes in the mash that degrade fatty acid hydroperoxides

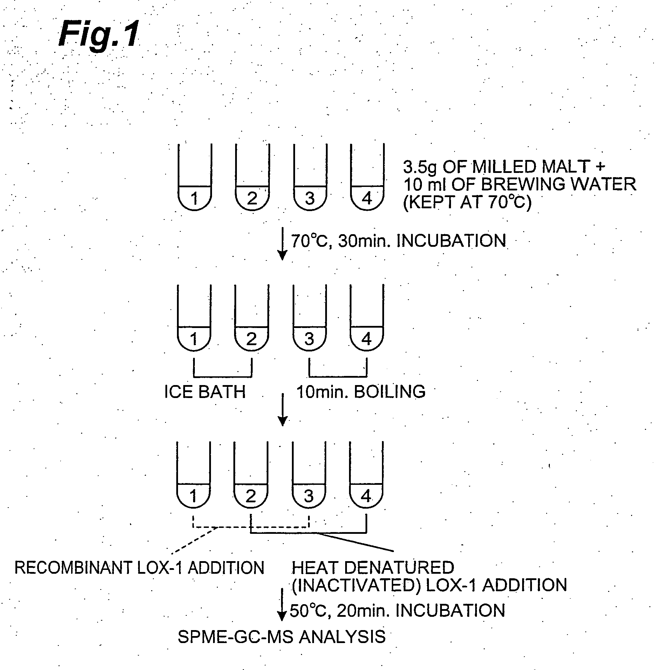

As shown in Fig. 1, four experimental numbers were set up and were subjected to the 3-step treatment described below. (1) As a. treatment to inactivate the lipoxygenase (LOX) in the malt, 3.5g of milled malt were added to 10 ml of brewing water kept at a temperature of 70°C in each of the experimental numbers, which were then incubated at 70°C for 30 minutes under stirring. The 1.6 units of LOX contained in the 3.5g of milled malt were inactivated with this treatment. (2) The fatty acid hydroperoxide lyases in the malt were treated in two ways: experimental numbers 1 and. 2 were placed in an ice bath to allow fatty acid hydroperoxide lyase activity to remain, and experimental numbers 3 and 4 were boiled for 10 minutes to inactivate the fatty acid hydroperoxide lyases. (3) As a pretreatment for assaying fatty acid hydroperoxide lyase activity, 1.6 units of recombinant barley lipoxygenase (hereinafter "recombinant LOX-1") were added to experimental numbers 1 and 3, while 1.6 units of heat-inactivated recombinant barley lipoxygenase (hereinafter "heat-denatured recombinant LOX-1") were added to experimental numbers 2 and 4. The recombinant LOX-1 and the heat-denatured recombinant LOX-1 were both prepared according to the method by Kuroda (Kuroda, H., Kobayashi, N., Kaneda, H., Watari, J., Takashio, M., J. Biosci. Bioengi., 93:, 2002). Then these samples were all incubated at 50°C for 20 minutes, and the supernatant was recovered by centrifugation (15,000 g, 10 minutes).

After the above 3-step treatment, the concentration of degradation products was measured by SPME-GC-MS (Hewlett Packard HP6890/ MSD system), with results hinting at the conclusions below. In these tests trans-2-nonenal was measured as the degradation product, with the measurement results expressed in nM units. In the present invention was measured the activity of 9-linoleic acid hydroperoxide lyase among fatty acid hydroperoxide lyases, since trans-2-nonenal is produced by the degradation of 9-linoleic acid hydroperoxide.

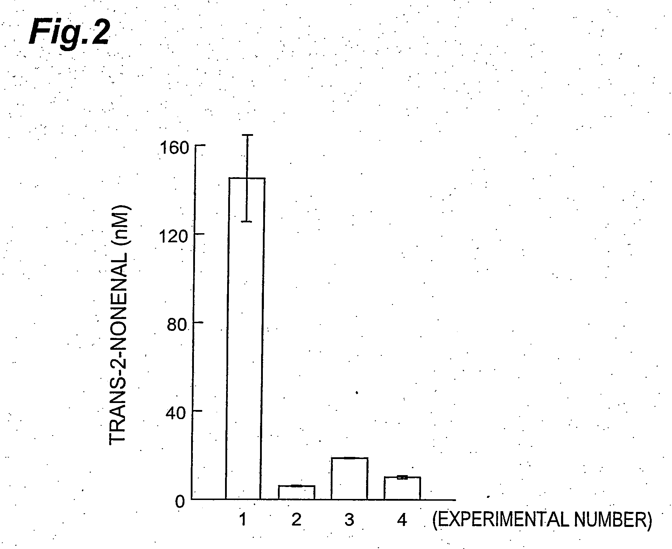

As shown in Fig. 2, the concentration of trans2-nonenal increased dramatically in the experimental number 1 where fatty acid hydroperoxide lyases were active and recombinant LOX-1 had been added. On the other hand, the concentration of trans-2-nonenal hardly increased in the experimental number 2, where fatty acid hydroperoxide lyases were active but heatdenatured recombinant LOX-1 had been added. In experimental numbers 3 and 4, where fatty acid hydroperoxide lyases had been inactivated, the concentration of trans-2-nonenal increased slightly through the addition of recombinant LOX-1 or heat-denatured recombinant LOX-1. These results suggest that there is a factor promoting the production of trans-2-nonenal that does not become inactivated by incubation at 70°C for 30 minutes but does when boiled. This factor is an enzyme (i.e. 9-linoleic acid hydroperoxide lyase, a fatty acid hydroperoxide lyase) that cleaves the 9-linoleic acid hydroperoxide generated in the oxidation of malt linoleic acid by LOX.

The above fatty acid hydroperoxide lyases possess thermal resistance in the manufacturing processes of malt-based sparkling beverages; therefore, the test below was carried out to verify their enzymatic action.

(Verification test 2)

Verification that fatty acid hydroperoxide lyases are thermally resistant enzymes that degrade fatty-acid hydroperoxides

A laboratory-level test by the congress method with mash separation over time was carried out to analyze the action of fatty acid hydroperoxide lyases during the brewing process. The mash samples were centrifuged (3,000 x g, 15 minutes) and the supernatant was separated from the precipitate; the latter was further extracted using an acetic acid buffer solution (0.1M, pH 6.0) containing 0.15% Triton X. Linoleic acid hydroperoxides (9-HPOD and 13-HPOD) were assayed as the fatty acid hydroperoxides in the present test. The rate of decrease of linoleic acid hydroperoxides was measured as follows.

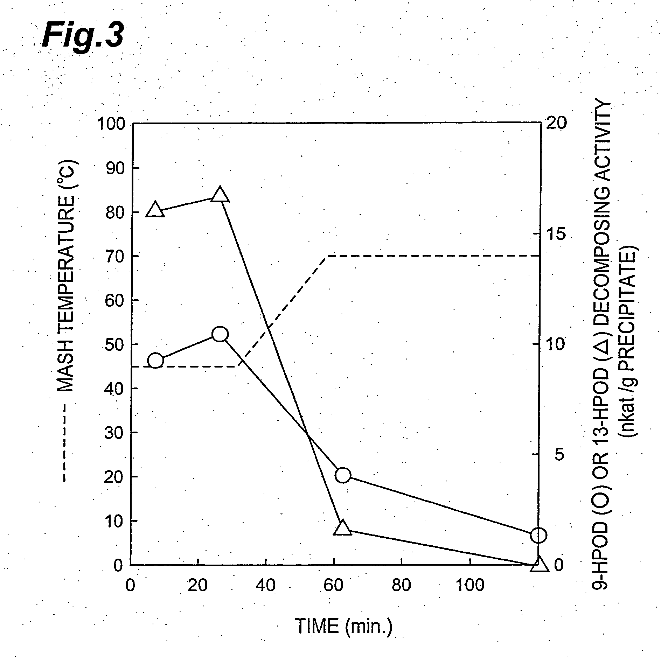

To a cuvette were added 500μl of acetic acid buffer solution (0.1M, pH 6.0) and 9-HPOD or 13-HPOD at a final concentration of 40μM, then 5 μl of enzyme solution (separated supernatant or precipitate extract) were added and the whole was mixed; next, the UV absorption at 234 nm of the conjugated dienes in 9-HPOD and 13-HPOD was monitored to determine the rate of decrease of the linoleic acid hydroperoxides. The enzyme activity (degradation activity (nkat/g of precipitate)) is obtained using the formula below based on the measurement result, as shown in Fig. 3. The measuring instrument used herein was a Hitachi spectrophotometer U-3500.

$$\text{Enzyme activity (nkat/g) = UV absorption decrease per}$$

$$\text{minute at 234 nm x 0.667 x total reaction solution}$$

$$\text{(mL)} \div \text{enzyme solution (mL)} \div \text{enzyme solution}$$

$$\text{concentration (g/mL)}$$

Though not in the mash supernatant, degradation activity was detected in an extract solubilized from the precipitate using surfactants. In the early stage of brewing, the degradation activity of 13-HPOD (Δ: open triangle) was roughly twice that of 9-HPOD (○: open circle). It is thus safe to affirm that 9-linoleic acid hydroperoxide lyase and 13-linoleic acid hydroperoxide lyase have a higher thermal resistance than lipoxygenase, as they both show enzyme activity even after 30 minutes at 70°C. Also, 9-linoleic acid hydroperoxide lyase and 13-linoleic acid hydroperoxide lyase have different thermal resistances, being higher for 9-linoleic acid hydroperoxide lyase, which shows still a residual activity of almost 10% of the maximum value (brewing early stage) after mashing is over. The .difference in degradation activity between 9-HPOD and 13-HPOD indicates that there are two linoleic acid hydroperoxide lyase isozymes with differing substrate specificity, which produce respectively nonenal (trans-2-nonenal) and hexanal.

Verification tests 1 and 2 prove that fatty acid hydroperoxide lyases (9-linoleic acid hydroperoxide lyase) are the factor behind the degradation products (trans-2-nonenal) from fatty acid hydroperoxides (9-linoleic acid hydroperoxide). Next, the test below was carried out in order to assess the influence of fatty acid hydroperoxide lyases on the final product (malt-based sparkling beverages).

(Verification test 3)

Verification that staleness substances in the post-fermentation product increase when fatty acid hydroperoxide lyases are active in the mash

In the test, the treatments (1) to (3) were identical to those of Verification test 1 but using herein different amounts of milled malt, brewing water and LOX in the four experimental numbers 1 to 4. (1) 17.5g of milled malt were added to 50 ml of brewing water kept at a temperature of 70°C in each of the experimental numbers, which were then incubated at 70°C for 30 minutes under stirring. Next experimental numbers 1 and 2 were placed in an ice bath and experimental numbers 3 and 4 were boiled for 10 minutes. (2) Experimental numbers 1 and 2 were placed in an ice bath to allow fatty acid hydroperoxide lyase activity to remain, and experimental numbers 3 and 4 were boiled for 10 minutes to inactivate the fatty acid hydroperoxide lyases. (3) Next, 7.9 units of recombinant LOX-1 were added to experimental numbers 1 and 3, and 7.9 units of heat-denatured recombinant LOX-1 were added to experimental numbers 2 and 4. The recombinant LOX-1 and the heat-denatured recombinant LOX-1 were both prepared similarly to Verification test 1. Thereafter, all experimental samples were incubated at 50°C for 20 minutes, and were filtered with filter paper.

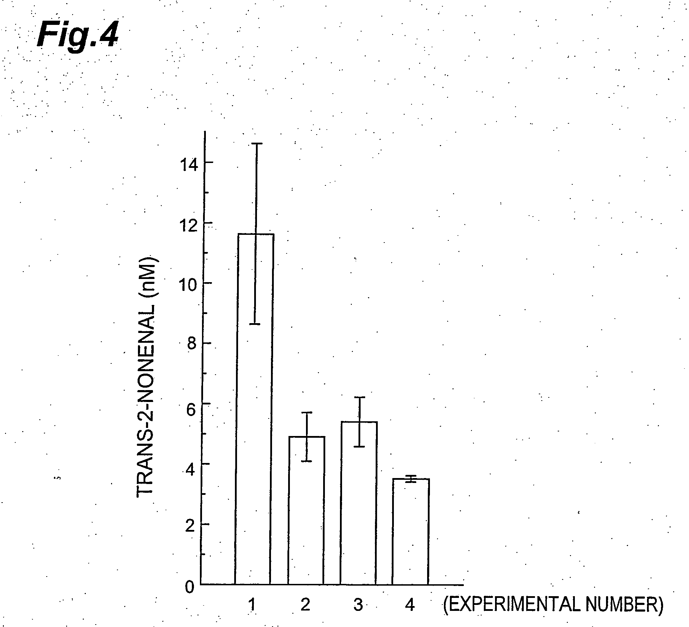

The remaining residue was further washed with 50ml of distilled water kept at 50°C. To this were added 0.2g of hop extract, then after boiling at 100°C for 90 minutes, 1.2g of bottom yeast were added and fermented at 12°C for 11 days. The fermentation liquid supernatant was matured at 12°C for 1 week and a further 2 weeks at 4°C, then it was filtered using a membrane filter and its trans-2-nonenal content was measured similarly to Verification test 1 (Fig. 4). The disparity in trans-2-nonenal concentration of Verification example 1 was observed also for this beer, proving thus that fatty acid hydroperoxide lyases (9-linoleic acid hydroperoxide lyase) are active in the brewing step, etc, and consequently, that the degradation products (trans-2-nonenal) generated thereby are also present in the final product.

In the experimental number 3, wherein fatty acid hydroperoxide lyases had been inactivated, the residual amount of trans-2-nonenal remaining in the final product was small, which proves that employing malt having low fatty acid hydroperoxide lyase activity in the manufacture of malt-based sparkling beverages is useful for decreasing stale flavor in the manufactured product. Measurement methods of fatty acid hydroperoxide lyase activity are described below by way of examples.

(Example 1)

Measurement of fatty acid hydroperoxide lyase activity by HPLC

1g of malt was milled with a coffee mill, then 10mL of acetic acid buffer solution (0.1M, pH 5.5) containing 0.15% Tween20 were added thereto and the whole was stirred for 1 hour. The mixture was centrifuged at 4°C and 15,000g, and the supernatant was separated. To 1mL of supernatant were added 9-HPOD or 13-HPOD at a final concentration of 100μM; after incubation for 15 minutes at 25°C, 1mL of an ethanol solution containing 0.1% 2,4-dinitrophenyl hydrazine and 0.5M acetic acid was added and mixed, for reaction quenching and derivatization. The solution was then allowed to stand at room temperature for 3 hours and was extracted in hexane. Nonenal, a generated degradation product, was separated and quantified by high performance liquid chromatography (C-R7A/LV-10A HPLC system) using a Zorbax ODS column. The eluent was acetonitrile/water/acetic acid (600:400:1). After the assay of the generated degradation product (nonenal), enzyme activity was calculated according to the following formula:

Enzyme activity (mU/g)= amount of degradation product

generated per minute (μM) x total reaction solution

(mL) ÷ enzyme solution (mL) ÷ enzyme solution

concentration (g/mL)

9-Linoleic acid hydroperoxide lyase activity was determined for 20 malt samples by the above method. The results showed that the 9-linoleic acid hydroperoxide lyase activity for each malt sample ranged roughly.from 2 to 6 mU/g.

(Example 2)

Assay of fatty acid hydroperoxide lyase activity by gas chromatography

Malt extract was prepared in the same way as in Example 1. 1mL of extract was injected in a vial for gas chromatography and was ice-chilled. 9-HPOD or 13-HPOD were added at a final concentration of 100μM, then after incubation at 25°C for 10 minutes, a Supelco polydimethylsiloxane SPME fiber was inserted, and after a further 5 minutes incubation, the sample was analyzed by gas chromatography (Hewlett Packard HP6890/MSD system). The conditions for the quantitative determination of hexanal / nonenal were: capillary column DB-1 by J&W (30m x 0.25mm, film thickness 1μm), helium as a carrier gas (1mL/minute), oven conditions from 60°C to 225°C (5°C/minute), and select ion mode (m/z: 70, 72). After the assay of the generated degradation product (aldehydes), enzyme activity was calculated according to the following formula:

Enzyme activity (mU/g)= amount of degradation product

generated per minute (μM) x total reaction solution

(mL) ÷ enzyme solution (mL) ÷ enzyme solution

concentration (g/mL).

9-Linoleic acid hydroperoxide lyase activity was determined for 20 malt sample by the above method. The results showed that the 9-linoleic acid hydroperoxide lyase activity for each malt sample ranged roughly from 2 to 6 mU/g.

(Example 3)

Assay of fatty acid hydroperoxide lyase activity by measuring decreased amount of substrate

Malt extract was prepared in the same way as in Example 1. To a cuvette were added 500μl of acetic acid buffer solution (0.1M, pH 6.0) and 9-HPOD or 13-HPOD at a final concentration of 40μM, then 5 μl of enzyme solution were added and the whole was mixed; next, the UV absorption at 234 nm of the conjugated dienes in 9-HPOD and 13-HPOD was monitored to determine the rate of decrease of the linoleic acid hydroperoxides. The measurement was carried out similarly to Verification test 2. Enzyme activity can be calculated from the decreasing rate of linoleic acid hydroperoxide using the following formula:

Enzyme activity (nkat/g) = UV absorption decrease per

minute at 234 nm x 0.667 x total reaction solution

(mL) ÷ enzyme solution (mL) ÷ enzyme solution

concentration (g/mL)

9-Linoleic acid hydroperoxide lyase activity was determined for 20 malt samples by the above method. The results showed that the 9-linoleic acid hydroperoxide lyase activity for each malt sample ranged roughly from 5 to 20 nkat/g.

(Example 4)

Correlation between staleness index and fatty acid hydroperoxide lyases

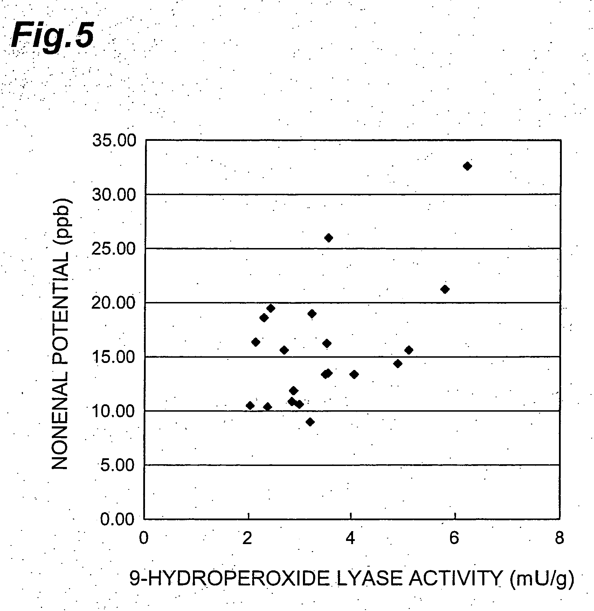

20 malt samples were provided for preparing wort by the congress method. The wort nonenal potential was measured by the method of Dorst et al., and the fatty acid hydroperoxide lyases of the malts were evaluated using the method of Example 2. Both variables were plotted together and revealed a positive correlation (r=0.53), as shown in Fig. 5. The nonenal potential is an indicator that allows predicting the staleness of the final product. Preferred malts herein are those with a low fatty acid hydroperoxide lyase activity, i.e. with a nonenal potential of 10 ppb or less, more preferably 1 ppb or less. Therefore, the evaluation of the fatty acid hydroperoxide lyases, which are correlated with wort nonenal potential, allows predicting the staleness of the final product.

**Industrial Applicability**

The screening method according to the present invention allows specifically screening malts in which the generation of staleness substances such as aldehydes, etc., caused by fatty acid hydroperoxide degradation is inhibited, affording thus a useful method for producing malt-based sparkling beverages having a reduced stale flavor. Using malts having low fatty acid hydroperoxide lyase activity as screened by the screening method of the present invention allows curbing the generation of degradation products (staleness substances) in the brewing step, etc., making it possible therefore to produce malt-based sparkling beverages having an excellent staleness resistance.

**Claims**

1. A method for screening malt comprising a step of evaluating fatty acid hydroperoxide lyase activity in malt.

2. A method for screening malt according to claim 1, wherein said fatty acid hydroperoxide lyase activity is evaluated by measuring the amount of the degradation products generated upon degradation of fatty acid hydroperoxides by said fatty acid hydroperoxide lyases.

3. A method for screening malt according to claim 1, wherein said fatty acid hydroperoxide lyase activity is evaluated by measuring the decreased amount of fatty acid hydroperoxides due to degradation of fatty acid hydroperoxides by said fatty acid hydroperoxide lyases.

4. A method for producing a malt-based sparkling beverage comprising a step of using malt having low fatty acid hydroperoxide lyase activity screened by a screening method according to any one of claims 1 to 3.

# Fig.1

3.5g OF MILLED MALT +
10 ml OF BREWING WATER
(KEPT AT 70°C)

70°C, 30min. INCUBATION

ICE BATH    10min. BOILING

RECOMBINANT LOX-1 ADDITION    HEAT DENATURED
(INACTIVATED) LOX-1 ADDITION

50°C, 20min. INCUBATION

SPME-GC-MS ANALYSIS

## Fig.2

# Fig.3

Fig.4

Fig.5

## INTERNATIONAL SEARCH REPORT

| | International application No. |
|---|---|
| | PCT/JP03/07887 |

**A. CLASSIFICATION OF SUBJECT MATTER**
Int.Cl$^7$ C12Q1/527, G01N33/50, G01N33/15, C12C1/16

According to International Patent Classification (IPC) or to both national classification and IPC

**B. FIELDS SEARCHED**

Minimum documentation searched (classification system followed by classification symbols)
Int.Cl$^7$ C12Q1/00-70, C12N9/00-99, G01N33/00-98, C12C1/00-13/10

Documentation searched other than minimum documentation to the extent that such documents are included in the fields searched

Electronic data base consulted during the international search (name of data base and, where practicable, search terms used)
MEDLINE(STN), WPI(STN), BIOSIS(STN), JICST-EPLUS(STN)

**C. DOCUMENTS CONSIDERED TO BE RELEVANT**

| Category* | Citation of document, with indication, where appropriate, of the relevant passages | Relevant to claim No. |
|---|---|---|
| P,Y | H. KURODA et al., "Characterization of factors involved in the production of 2(E)-nonenal during mashing"., Bioscience Biotechnology and Biochemistry, April, 2003, Vol.67, No.4, pages 691 to 697 | 1-4 |
| A | JP 2001-029097 A (Sapporo Breweries Ltd.), 06 February, 2001 (06.02.01), (Family: none) | 1-4 |

☐ Further documents are listed in the continuation of Box C.    ☐ See patent family annex.

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>07 August, 2003 (07.08.03) | Date of mailing of the international search report<br>19 August, 2003 (19.08.03) |
| Name and mailing address of the ISA/<br>Japanese Patent Office | Authorized officer |
| Facsimile No. | Telephone No. |

Form PCT/ISA/210 (second sheet) (July 1998)

15